# EUROPEAN PATENT SPECIFICATION

(11) **EP 4 179 989 B1**
(45) Date of publication and mention of the grant of the patent: **03.09.2025**
(21) Application number: 22206307.5
(22) Date of filing: 09.11.2022
(51) Int. Cl.: A61B 18/14, A61B 5/00, A61B 5/06

(54) **ESTIMATING CONTACT FORCE APPLIED BETWEEN CATHETER AND TISSUE USING TRANSMITTER AND RECEIVERS OF THE CATHETER**
SCHÄTZUNG DER ZWISCHEN KATHETER UND GEWEBE ANGEWANDTEN KONTAKTKRAFT UNTER VERWENDUNG VON SENDER UND EMPFÄNGERN DES KATHETERS
ESTIMATION DE LA FORCE DE CONTACT APPLIQUÉE ENTRE UN CATHÉTER ET UN TISSU À L'AIDE D'UN ÉMETTEUR ET DE RÉCEPTEURS DU CATHÉTER

(30) Priority: 10.11.2021 US 202117523155
(43) Date of publication of application: 17.05.2023
(73) Proprietor: Biosense Webster (Israel) Ltd., Yokneam 2066717 (IL)
(72) Inventor: GOVARI, Assaf, 2066717 Yokneam (IL); BEECKLER, Christopher Thomas, Irvine, 92618 (US); ALTMANN, Andres Claudio, 2066717 Yokneam (IL); GLINER, Vadim, 2066717 Yokneam (IL); KEYES, Joseph Thomas, Irvine, 92618 (US)
(74) Representative: Carpmaels & Ransford LLP

(56) References cited:
- EP-A1- 3 785 612
- US-A1- 2009 093 806
- US-A1- 2017 164 999
- US-A1- 2020 155 224

## Description

### FIELD OF THE INVENTION

The present invention relates generally to medical devices, and particularly to methods and systems for estimating contact force applied between catheter and tissue.

### BACKGROUND OF THE INVENTION

Various techniques for measuring and/or estimating contact force between a medical device and tissue and relative positions thereof are known in the art.

For example, U.S. Patent Application Publication 2020/0206461 describes a system that includes an expandable distal-end assembly, a proximal position sensor, a distal position sensor, and a processor. The expandable distal-end assembly is coupled to a distal end of a shaft for insertion into a cavity of an organ of a patient. The proximal and distal position sensors are located at a proximal end and a distal end of the distal-end assembly, respectively. The processor is configured to estimate a position and a longitudinal direction of the proximal sensor, and a position of the distal sensor, all in a coordinate system used by the processor. The processor is further configured to project the estimated position of the distal sensor on an axis defined by the estimated longitudinal direction, and calculate an elongation of the distal-end assembly by calculating a distance between the estimated position of the proximal sensor and the projected position of the distal sensor.

U.S. Patent Application Publication 2018/0076336 describes systems, devices and methods that integrate stretchable or flexible circuitry, including arrays of active devices for enhanced sensing, diagnostic, and therapeutic capabilities. The invention enables conformal sensing contact with tissues of interest, such as the inner wall of a lumen, the brain, or the surface of the heart. Such direct, conformal contact increases accuracy of measurement and delivery of therapy. Further, the invention enables the incorporation of both sensing and therapeutic devices on the same substrate allowing for faster treatment of diseased tissue and fewer devices to perform the same procedure. Other catheter systems are described in US2009/093806, EP3785612 and US2017/164999.

### SUMMARY OF THE INVENTION

An embodiment of the present invention that is described herein provides a system including a catheter and a processor. The catheter includes an expandable distal-end assembly (EDEA) having: (i) a transmitter, which is coupled to the EDEA and is configured to transmit a first signal, and (ii) one or more receivers, which are coupled to an elastic component of the EDEA, and are configured to produce one or more respective second signals in response to receiving the first signal. The processor is configured to estimate, based on the one or more respective second signals, a force applied to the elastic component.

In some embodiments, the EDEA includes a basket, the elastic component includes one or more splines of the basket, the force includes a contact force applied between the EDEA and tissue of an organ, and in response to applying the contact force, at least one of the splines is configured to deform for conforming with a shape of the tissue. In other embodiments, the transmitter is coupled to a rigid component of the EDEA. In yet other embodiments, the rigid component includes an irrigation apparatus or a shaft of the catheter.

In an embodiment, the processor is configured to: (i) hold a calibration dataset for quantifying a relation between at least one of the respective second signals and a level of deformation of at least one of the splines, and (ii) estimate the contact force based on at least one of the respective second signals and the calibration set. In another embodiment, the processor is configured to produce the calibration set before insertion of the EDEA into the organ, and to estimate the contact force when the EDEA is placed in contact with the tissue. In yet another embodiment, when the EDEA is in an expanded position, the one or more respective second signals include: (i) a first given signal received from a given receiver of the one or more receivers without applying the contact force, and (ii) a second given signal received from the given receiver when the contact force is applied, and the processor is configured to estimate the contact force based on the first and second given signals.

In some embodiments, the EDEA includes a balloon, and the elastic component includes a member of the balloon. In other embodiments, the force includes a contact force applied between the member and tissue of an organ, and in response to applying the contact force, the member is configured to deform for conforming with a shape of the tissue, and the processor is configured to: (i) hold a calibration dataset for quantifying a relation between at least one of the respective second signals and a level of deformation of at least a section of the member, and (ii) estimate the contact force based on at least one of the respective second signals and the calibration set.

There is additionally provided in the disclosure a method which is not part of the claimed invention, the method including inserting into an organ of a patient a catheter including an expandable distal-end assembly (EDEA) having: (i) a transmitter, which is coupled to the EDEA for transmitting a first signal, and (ii) one or more receivers, which are coupled to an elastic component of the EDEA for producing one or more respective second signals in response to receiving the first signal. The first signal is applied to the transmitter and the one or more respective second signals are received. Based on the one or more respective second signals, a force applied to the elastic component is estimated.

There is further provided, in accordance with an embodiment of the present invention, a method for producing a catheter, the method includes coupling, to an expandable distal-end assembly (EDEA) of a catheter, a transmitter for transmitting a first signal. One or more receivers for producing one or more respective second signals in response to receiving the first signal, are coupled to an elastic component of the EDEA. At least the one or more receivers are electrically coupled to a processor for receiving the one or more respective second signals and for estimating, based on the one or more respective second signals, a force applied to the elastic component.

In some embodiments, the EDEA includes a basket, the elastic component includes one or more splines of the basket, the force includes a contact force applied between the EDEA and tissue of an organ, and in response to applying the contact force, at least one of the splines is configured to deform for conforming with a shape of the tissue. In other embodiments, the transmitter is coupled to a rigid component of the EDEA, and the rigid component includes a shaft or an irrigation apparatus of the catheter. In yet other embodiments, coupling the one or more receivers includes coupling a given receiver to a given spline of the basket.

In an embodiment, the EDEA includes a balloon, and the elastic component includes a member of the balloon. In another embodiment, the force includes a contact force applied between the member and tissue of an organ, in response to applying the contact force, the member is configured to deform for conforming with a shape of the tissue.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based position-tracking and ablation system, in accordance with an embodiment of the present invention;
Fig. 2 is a schematic, pictorial illustration of a distal-end assembly of a catheter of the system depicted in Fig. 1, in accordance with an embodiment of the present invention;
Fig. 3 is a flow chart that schematically illustrates a method for estimating a contact force applied between the distal-end assembly and tissue of a patient heart, in accordance with an embodiment of the present invention; and
Fig. 4 is a flow chart that schematically illustrates a method for producing the distal-end assembly depicted in Fig. 2, in accordance with an embodiment of the present invention.

### DETAILED DESCRIPTION OF EMBODIMENTS

### OVERVIEW

Some medical procedures, such as radiofrequency (RF) ablation of tissue in a patient heart, require controlled contact force between ablation electrodes of an ablation catheter inserted into a patient heart and the heart tissue intended to be ablated by applying RF signals to the ablation electrodes. The ablation electrodes may be coupled to flexible splines of a basket-shaped expandable distal-end assembly. During the ablation procedure the distal-end assembly is inserted into the heart and expanded to an expanded position for exposing the ablation electrodes to the tissue. Some of the splines are placed in contact with the tissue and are deformed in order to conform with the tissue and for placing the electrodes in contact with the tissue intended to be ablated. The controlled contact force between the respective splines and the tissue is important for properly ablating the tissue and for producing, in the tissue in question, a lesion having specified properties.

In principle, it is possible to couple to the distal-end assembly, a device for measuring the contact force. For example, the device may include one or more piezoelectric crystals that output voltage in response to sensing contact force applied to the splines. However, the piezoelectric crystals may increase the complexity of the catheter and may provide insufficient accuracy of the measured or estimated contact force, *inter alia,* due to the flexibility of the splines.

Embodiments of the present invention that are described hereinbelow provide improved techniques for estimating the contact force applied, during an ablation procedure, between tissue in question (e.g., of a patient heart) and an expandable distal-end assembly (EDEA) of a catheter. Note that the improved techniques rely on quantifying and using the elastic properties of a flexible component of the distal-end assembly.

In some embodiments, a system for performing RF ablation to tissue in question comprises a catheter and a processor. The catheter comprises an EDEA having a transmitter and one or more receivers, typically implemented in electrical coils. The transmitter is coupled to a rigid component of the EDEA, e.g., a shaft or an irrigation apparatus of the catheter. The one or more receivers are coupled to respective elastic component(s) of the EDEA. For example, (i) in an EDEA comprising a basket, one or more receiver(s) may be coupled to each spline of the basket, and (ii) in an EDEA comprising a balloon, the one or more receivers may be disposed and fitted at several positions on the outer surface of the balloon.

In some embodiments, the one or more receivers are electrically connected to the processor and the transmitter is electrically connected to the processor or to a pulse generator controlled by the processor. In some embodiments, the electrical connection may be carried out using (i) electrical wires or traces running between the proximal and distal ends of the catheter, e.g., between the EDEA and an operating console of the system that comprises the processor, or (ii) wirelessly, using wireless devices coupled to the proximal and distal ends of the catheter.

In some embodiments, the processor is configured to apply to the transmitter a transmission signal, referred to herein as a first signal, and at least one of the receivers is configured to produce a receiving signal, referred to herein as a second signal, in response to receiving the first signal.

In some embodiments, during the ablation procedure, a physician inserts the EDEA into the patient heart, expands the EDEA to an expanded position, places the EDEA in contact with the tissue and applies contact force to the elastic component (e.g., splines) for performing the ablation. The processor is configured to estimate, based on the second signal received from the receiver, the contact force applied to the respective one or more splines. Note that the intensity of the first signal sensed by the receiver is proportional to the distance between the transmitter and the respective receiver. In some embodiments, in response to the contact force applied by the physician, a given spline of the splines deforms, and the deformation is sensed by the altered intensity of the first signal sensed by the respective receiver.

In some embodiments, the processor is configured to hold a calibration dataset (e.g., a calibration table) for quantifying the relation between the one or more second signals and the level of deformation of the one or more respective splines. The calibration process may be carried out as part of the manufacturing process of the EDEA, or before inserting the catheter into the patient heart.

In other embodiments, after expanding the EDEA and before placing the EDEA in contact with the heart tissue, the physician may control the processor to: (i) apply the first signal using the transmitter, and (ii) receive the one or more second signal(s) for obtaining a reference before applying the contact force to the splines. In such embodiments, after placing the EDEA in contact with the tissue and applying the contact force to the splines, the processor is configured to reapply the first signal and to receive an additional second signal. The processor is configured to estimate the contact force applied to the respective splines using the received second signals before and after applying the contact force to the splines.

The disclosed techniques improve the quality of RF ablation procedures by improving the accuracy and reducing the complexity of sensing contact force applied between ablation electrodes and tissue intended to be ablated during the RF ablation procedure. The disclosed techniques are also applicable, *mutatis mutandis,* to any other medical procedure that requires accurate sensing of contact force between a medical device pressed against tissue.

### SYSTEM DESCRIPTION

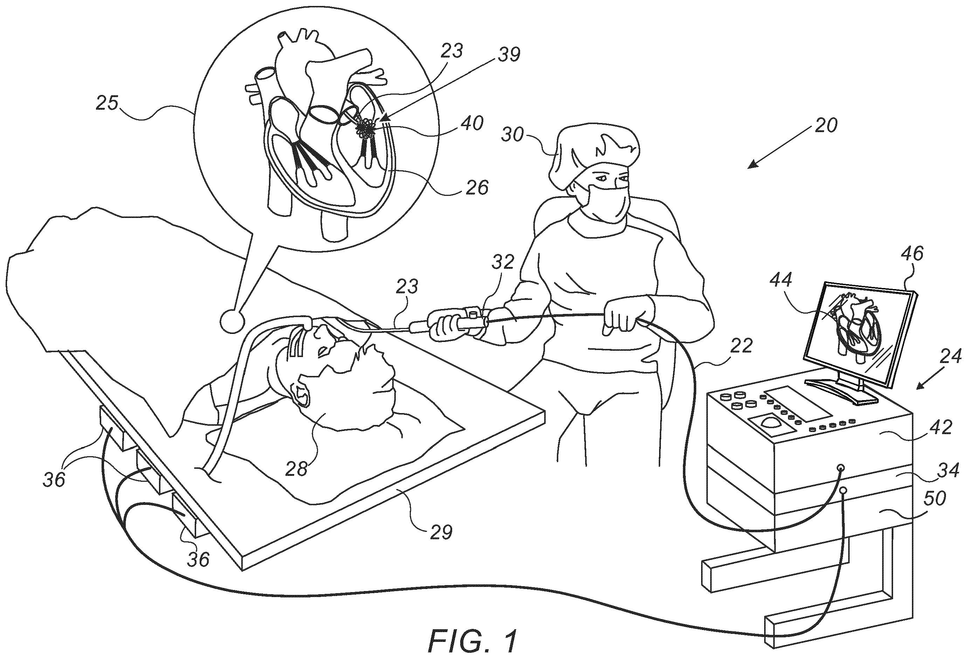

Fig. 1 is a schematic, pictorial illustration of a catheter-based position-tracking and ablation system 20, in accordance with an embodiment of the present invention. In some embodiments, system 20 comprises a catheter 22, in the present example an expandable cardiac catheter, and a control console 24. In the embodiment described herein, catheter 22 may be used for any suitable therapeutic and/or diagnostic purposes, such as ablation of tissue in a heart 26 and for mapping cardiac arrhythmias by sensing intra-cardiac electrical signals.

In some embodiments, console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system, and is configured to store data for the software in a memory 50. The software may be downloaded to console 24 in electronic form, over a network, for example, or it may be provided on non-transitory tangible media, such as optical, magnetic or electronic memory media. Alternatively, some or all of the functions of processor 42 may be carried out using an application-specific integrated circuit (ASIC) or any suitable type of programmable digital hardware components.

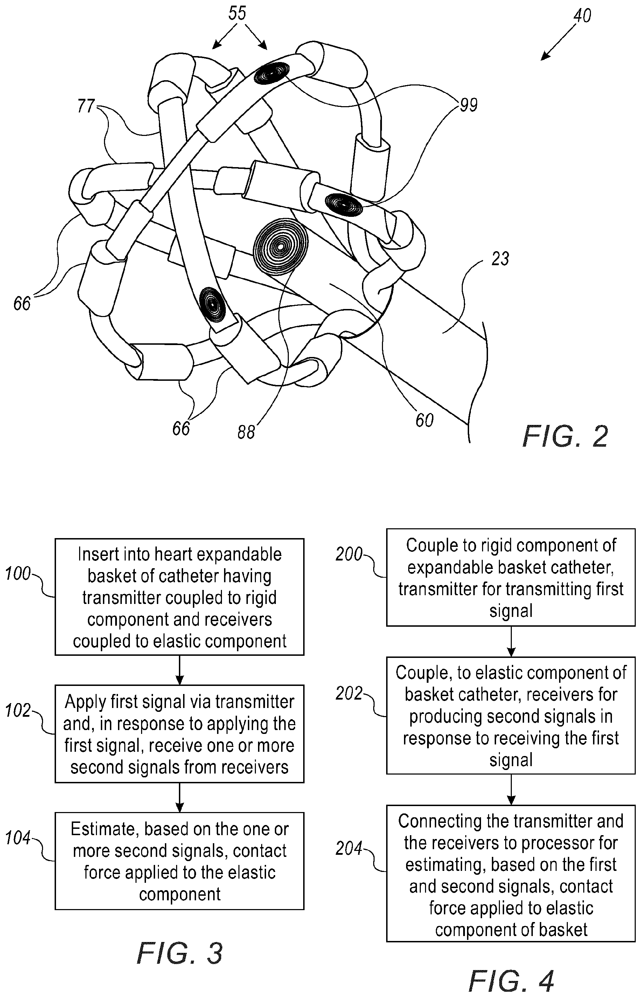

Reference is now made to an inset 25. In some embodiments, catheter 22 comprises an expandable distal-end assembly 40 having multiple splines (shown in detail in Fig. 2 below), and a shaft 23 for inserting distal-end assembly 40 to a target location for ablating tissue in heart 26. During an ablation procedure, physician 30 inserts catheter 22 through the vasculature system of a patient 28 lying on a table 29. Physician 30 moves distal-end assembly 40 to the target location in heart 26 using a manipulator 32 near a proximal end of catheter 22, which is connected to interface circuitry of processor 42.

In some embodiments, catheter 22 comprises a position sensor 39 of a position tracking system, which is coupled to the distal end of catheter 22, e.g., in close proximity to distal-end assembly 40. In the present example, position sensor 39 comprises a magnetic position sensor, but in other embodiments, any other suitable type of position sensor (e.g., other than magnetic-based) may be used.

Reference is now made back to the general view of Fig. 1. In some embodiments, during the navigation of distal-end assembly 40 in heart 26, processor 42 receives signals from magnetic position sensor 39 in response to magnetic fields from external field generators 36, for example, for the purpose of measuring the position of distal-end assembly 40 in heart 26. In some embodiments, console 24 comprises a driver circuit 34, configured to drive magnetic field generators 36. Magnetic field generators 36 are placed at known positions external to patient 28, e.g., below table 29.

In some embodiments, processor 42 is configured to display, e.g., on a display 46 of console 24, the tracked position of distal-end assembly 40 overlaid on an image 44 of heart 26.

The method of position sensing using external magnetic fields is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

This particular configuration of system 20 is shown by way of example, in order to illustrate certain problems that are addressed by embodiments of the present invention and to demonstrate the application of these embodiments in enhancing the performance of such a system. Embodiments of the present invention, however, are by no means limited to this specific sort of example system, and the principles described herein may similarly be applied to other sorts of medical systems and procedures.

### DISTAL-END ASSEMBLY HAVING TRANSMITTER AND RECEIVERS FOR MEASURING CONTACT FORCE

Fig. 2 is a schematic, pictorial illustration of distal-end assembly 40 in an expanded position, in accordance with an embodiment of the present invention.

In some embodiments, distal-end assembly 40 comprises multiple splines 55 of a basket-shaped assembly. Each spline 55 comprises a flexible arm 77 made from a suitable elastic substance, such as but not limited to a flexible printed circuit board (PCB), or a suitable biocompatible and flexible metal alloy (e.g., nickel-titanium based, such as nitinol) that may be partially coated with one or more electrically insulating layer(s) to prevent electrical shorts between splines 55.

In some embodiments, one or more ablation electrodes 66 are coupled to each spline 55 and are configured to apply ablation pulse (s) to tissue of heart 26. The ablation pulse(s) are intended to kill cells of the tissue in question and to produce, instead of the tissue, a lesion that prevents or reduces the propagation of electrophysiological (EP) waves through the ablated tissue.

In some embodiments, distal-end assembly 40 comprises a transmitter 88 and one or more receivers 99. Transmitter 88 is coupled to a rigid component of distal-end assembly 40, in the present example, transmitter 88 is coupled to an irrigation apparatus, also referred to herein as an irrigator 60, which is configured to apply irrigation fluid when applying the ablation signals to tissue of heart 26, or at any other suitable time interval of the ablation procedure. In other embodiments, transmitter 88 may be coupled to any other suitable rigid component of distal-end assembly 40, such as shaft 23. In the context of the present disclosure and in the claims, the term "rigid" refers to a component of catheter 22 that moves together with the catheter and whose location is not affected by any force, such as contact force, applied to distal-end assembly 40, as will be described in detail below. In other words, transmitter 88 has the same angular velocity and the same angular acceleration of the distal end of shaft 23.

In some embodiments, one or more receivers 99 are coupled to respective elastic components of distal-end assembly 40. In the present example, distal-end assembly 40 comprises a basket having multiple splines 55, and one receiver 99 is coupled to each spline 55 of the basket. In alternative embodiments, distal-end assembly 40 may comprise a balloon (not shown) having a flexible member, or any other suitable type of expandable distal-end assembly. In case of a balloon, receivers 99 may be disposed at several positions on the outer surface of the balloon. Note that in the example of Fig. 2, only three receivers 99 are shown, but a receiver 99 is coupled to each spline 55 even though some of receivers 99 are hidden by the isometric perspective of distal-end assembly 40, and therefore, are not shown in the example configuration of Fig. 2.

In some embodiments, transmitter 88 and receivers 99 may be implemented using electrical coils that are coupled to irrigator 60 and splines 55, respectively. The coils of transmitter 88 and receivers 99 are electrically connected to console 24, and more particularly, to processor 42 using any suitable connection techniques described in detail below.

In some embodiments, receivers 99 are electrically connected (e.g., via traces of the aforementioned flexible PCB, and via catheter 22) to processor 42. Moreover, transmitter 88 is electrically connected to processor 42 (e.g., via wires running between (i) the distal end of shaft 23 or irrigator 60, and (ii) the proximal end of catheter 22). Additionally, or alternatively, transmitter 88 may be electrically connected to a pulse generator (not shown) controlled by processor 42 and configured to apply one or more pulses using transmitter 88 as will be described below.

In other embodiments, the electrical connection between (i) transmitter 88 and/or receivers 99 and (ii) processor 42, may be carried out using wireless devices (not shown) coupled to the proximal and distal ends of catheter 22.

In some embodiments, processor 42 is configured to control transmitter 88 to apply a transmission signal, also referred to herein as a first signal. In response to receiving the first signal, at least one of and typically all receivers 99, are configured to produce a receiving signal, also referred to herein as a second signal.

In some embodiments, the second signal produced by a given receiver 99 is indicative of the distance between transmitter 88 and given receiver 99, as will be described below.

In some embodiments, during the ablation procedure, physician 30 inserts distal-end assembly 40 into a cavity of heart 26. Subsequently, physician 30 expands distal-end assembly 40 to an expanded position (as shown in the example of Fig. 2), places distal-end assembly 40 in contact with the tissue intended to be ablated, and applies contact force to splines 55 (or any elastic component of another sort of expandable distal-end assembly) for performing the ablation of the tissue in heart 26.

In some embodiments, processor 42 is configured to receive the second signals from receivers 99 and to estimate, based on the second signals, the contact force applied to the respective one or more splines 55. Note that the intensity of the first signal sensed by a given receiver 99 is indicative of (e.g., proportional to) the distance between transmitter 88 and given receiver 99, the smaller the distance the larger the intensity of the first signal sensed by given receiver 99. In the present example, the sensitivity of the estimated change in distance between transmitter 88 and given receiver 99 is about 0.01 mm, whereas when applying the contact force to distal-end assembly 40 the change in distance between transmitter 88 and given receiver 99 is typically larger than about 0.1 mm.

In the context of the present disclosure and in the claims, the terms "about" or "approximately" for any numerical values or ranges indicate a suitable dimensional tolerance that allows the part or collection of components to function for its intended purpose as described herein.

In some embodiments, in response to the contact force applied to distal-end assembly 40 by physician 30, arm 77 of a given spline 55 having given receiver 99 coupled thereto is deformed, and the deformation is sensed by the altered intensity of the first signal sensed by the given receiver 99.

In some embodiments, processor 42 is configured to hold a calibration dataset (e.g., a calibration table) for quantifying the relation between the one or more second signals and the level of deformation of the one or more respective splines 55. The calibration process may be carried out as part of the manufacturing process of distal-end assembly 40, or at any other time interval before inserting distal-end assembly 40 into heart 26 of patient 28.

In some embodiments, processor 42 is configured to translate the change in distance between transmitter 88 and given receiver 99 to the contact force applied to the spline having the given receiver coupled thereto (e.g., using the calibration dataset. As described above, the sensitivity of estimating the change in distance is higher than the typical deformation of the spline. Therefore, processor 42 is configured to estimate the contact force applied to the spline with a typical sensitivity of about 5 grams also referred to herein as gram-force (GF), whereas a typical value of contact force applied in such procedures is between about 0 grams and 100 grams.

In other embodiments, after expanding distal-end assembly 40 and before placing distal-end assembly 40 in contact with the tissue in question of heart 26, physician 30 may control processor 42 to: (i) apply the first signal using transmitter 88, and (ii) receive the one or more second signal(s) from one or more respective receivers 99 for obtaining a reference before applying the contact force to splines 55. In such embodiments, after placing distal-end assembly 40 in contact with the tissue intended to be ablated, and applying the contact force to splines 55, processor 42 is configured to control transmitter 88 to reapply the first signal. Subsequently, processor 42 is configured to receive from one or more receivers 99, additional second signals.

In some embodiments, processor 42 is configured to estimate the contact force applied to the respective splines 55 using the received second signals before and after applying the contact force to the respective splines 55.

In some embodiments, processor 42 holds a threshold indicative of the minimal level of contact force, which is applicable for applying the ablation pulse(s) to the tissue in question of heart 26. In some embodiments, processor 42 controls a RF pulse generator (not shown) of system 20 to apply the RF ablation signal(s) to a given ablation electrode 66, only when the contact force estimated by processor 42 using the techniques described above, is larger than the threshold. Thus, the disclosed techniques improve the quality of RF ablation procedures by improving the accuracy of the estimated contact force applied between ablation electrodes 66 and the tissue of heart 26, which is intended to be ablated during the RF ablation procedure described above. The disclosed techniques are also applicable, *mutatis mutandis,* to any other medical procedure that requires accurate and stable sensing of contact force between a medical device pressed against tissue.

The configuration of distal-end assembly 40 is provided in Fig. 2 by way of example, in order to illustrate certain problems that are addressed by embodiments of the present invention and to demonstrate the application of these embodiments in enhancing the performance of such a distal end for treating arrhythmias in patient heart. Embodiments of the present invention, however, are by no means limited to this specific sort of example distal-end assembly, and the principles described herein may similarly be applied to other sorts of catheters used in any suitable sort of medical systems and procedures that require measurement or estimation of contact force applied to any suitable type of medical device pressed against any suitable tissue of a patient.

Fig. 3 is a flow chart that schematically illustrates a method for estimating contact force applied between distal-end assembly 40 and tissue of heart 26, in accordance with an embodiment of the present invention.

The method begins at a catheter insertion step 100, with physician 30 inserting distal-end assembly 40 of catheter 22 into a cavity of heart 26. In some embodiments, distal-end assembly 40 comprises a basket having splines 55 and ablation electrodes 66 coupled to arms 77 of splines 55.

In some embodiments, distal-end assembly 40 has transmitter 88 coupled to a rigid component of distal-end assembly 40, in the present example, irrigator 60 or the distal end of shaft 23. Distal-end assembly 40 further comprises one or more receivers 99 coupled to at least one of and typically all splines 55, which are elastic components of distal-end assembly 40 and are configured to deform in response to applying contact force between the tissue and the respective spline(s) 55, as described in Fig. 2 above.

In some embodiments, transmitter 88 is controlled by processor 42 and is configured to apply a transmitted signal, also referred to herein as the first signal, as described in Fig. 2 above. Receivers 99 are configured to produce respective second signals in response to sensing/receiving the first signal applied by transmitter 88, as also described in Fig. 2 above.

At a signal application step 102, processor 42 controls transmitter 88 to apply the first signal, and, in response to applying the first signal, processor 42 receives one or more second signals from respective receivers 99, as described in Fig. 2 above.

At a contact force estimation step 104 that concludes the method, processor 42 estimates the contact force applied to the respective one or more splines 55, based on the one or more second signals received from the one or more respective receivers 99 coupled to the one or more respective splines 55 of distal-end assembly 40.

In some embodiments, a calibration process is carried out before estimating the contact force. In the calibration process, the applied contact force and resulting deformation of splines 55 are quantified relative to the second signal produced by one or more receivers 99 coupled to one or more splines 55. In other words, after the calibration, processor 42 is configured to receive from a given receiver 99 that is coupled to a given spline 55, a given second signal, and based on the given second signal, processor 42 is configured to provide physician 30 with an estimated change in the contact force applied to given spline 55 pressed against tissue of heart 26. The calibration may be carried out in the production of distal-end assembly 40, or at any other suitable time interval before applying the contact force to one or more splines 55 of distal-end assembly 40.

Fig. 4 is a flow chart that schematically illustrates a method for producing distal-end assembly 40 depicted in Fig. 2 above, in accordance with an embodiment of the present invention.

The method begins at a transmitter coupling step 200, with coupling transmitter 88 to a rigid component of catheter 22 and/or distal-end assembly 40. In the example of Fig. 2 above, the rigid component comprises an irrigation apparatus, referred to herein as irrigator 60, or the distal end of shaft 23. In other embodiments, transmitter 88 may be coupled to any other suitable rigid component of catheter 22.

In some embodiments, transmitter 88 is controlled by processor 42 and is configured to produce a first signal, as described in Fig. 2 above.

At a receiver coupling step 202, one or more receivers 99 are coupled to one or more respective elastic components (e.g., splines 55) of distal-end assembly 40. In some embodiments, in response to receiving or sensing the first signal applied by transmitter 88, receivers 99 are configured to produce a second signal indicative of the distance between transmitter 88 and the respective receiver 99.

In some embodiments, any suitable number of receivers 99 may be coupled along each spline 55, the number of coupled receivers 99 may be similar among all splines 55 or may differ between different splines 55. In one example implementation of distal-end assembly 40, one receiver 99 may be coupled to each spline 55. In another example implementation of distal-end assembly 40, multiple receivers 55 are coupled to a first spline 55, only one receiver 99 is coupled to a second spline 55, and a third spline 55 may not have any receiver 99.

At a connecting step 204 that concludes the method, transmitter 88 and receivers 99 are electrically connected to processor 42 for estimating, based on the first and second signals, the contact force applied to each spline 55 of distal-end assembly 40. The electrical connectors may comprise electrical leads or wires coupled between the distal and proximal ends of catheter 22, or electrical traces of the flexible PCB, or wireless devices coupled to the distal and proximal ends of catheter 22, as described in Fig. 2 above.

In some embodiments, after the production method may comprise additional steps, such as but not limited to: (i) coupling ablation electrodes to arms 77 of splines 55, (ii) coating one or more sections of one or more splines 55 using one or more electrically insulating layer(s), and (iii) coupling distal-end assembly 40 to catheter 22, for example by coupling splines 55 to the distal end of shaft 23 or to any other suitable component of catheter 22.

Although the embodiments described herein mainly address estimation of contact force applied between an expandable distal-end assembly of an RF ablation catheter and tissue intended to be ablated, the methods and systems described herein can also be used in other applications, such as in any application that requires accurate measurement of contact force applied between any suitable medical device and any suitable tissue of an organ of a patient. For example, in organs of an ear-nose-throat (ENT) system of a patient.

## Claims

1. A system (20), comprising:
a catheter (22), comprising an expandable distal-end assembly (EDEA) (40) having: (i) a transmitter (88), which is coupled to the EDEA (40) and is configured to transmit a first signal, and (ii) a plurality of receivers (99), which are coupled to an elastic component of the EDEA (40), and are configured to produce one or more respective second signals in response to receiving the first signal;
wherein one of the following conditions is satisfied:
(i) the EDEA (40) comprises a basket and one or more of the plurality of receivers (99) are coupled to each spline (55) of the basket; or
(ii) the EDEA (40) comprises a balloon and the plurality of receivers (99) are disposed at several positions on the outer surface of the balloon;
and wherein the system further comprises:
a processor (42), which is configured to: (i) hold a calibration dataset for quantifying a relation between at least one of the respective second signals and a level of deformation of at least one of the splines (55) or a member of the balloon, and (ii) estimate the contact force based on at least one of the respective second signals and the calibration set.

2. The system (20) according to claim 1 part (i), wherein the force comprises a contact force applied between the EDEA (40) and tissue of an organ, and wherein, in response to applying the contact force, at least one of the splines (55) is configured to deform for conforming with a shape of the tissue.

3. The system (40) according to claim 2, wherein the transmitter (88) is coupled to a rigid component of the EDEA (40).

4. The system (40) according to claim 3, wherein the rigid component comprises an irrigation apparatus (60) or a shaft of the catheter.

5. The system (20) according to claim 1 part (i), wherein the processor (42) is configured to produce the calibration set before insertion of the EDEA (40) into the organ, and to estimate the contact force when the EDEA (40) is placed in contact with the tissue.

6. The system (20) according to claim 2, wherein, when the EDEA (40) is in an expanded position, the one or more respective second signals comprise: (i) a first given signal received from a given receiver (99) of the one or more receivers (99) without applying the contact force, and (ii) a second given signal received from the given receiver (99) when the contact force is applied, and wherein the processor is configured to estimate the contact force based on the first and second given signals.

7. The system (20) according to claim 1 part (ii) wherein the elastic component comprises a member of the balloon.

8. A method for producing a catheter (22), the method comprising:
coupling, to an expandable distal-end assembly (EDEA) (40) of a catheter (22), a transmitter (88) for transmitting a first signal;
coupling, to an elastic component of the EDEA, a plurality of receivers (99) for producing one or more respective second signals in response to receiving the first signal; and
electrically coupling the plurality of receivers (99) to a processor (42) for receiving the one or more respective second signals and for estimating, based on the one or more respective second signals, a force applied to the elastic component;
wherein one of the following conditions is satisfied:
(i) the EDEA (42) comprises a basket and one or more of the plurality of receivers (99) are coupled to each spline (55) of the basket; or
(ii) the EDEA comprises a balloon and the plurality of receivers (99) are disposed at several positions on the outer surface of the balloon.

9. The method according to claim 8 part (i), wherein the transmitter (88) is coupled to a rigid component of the EDEA, and wherein the rigid component comprises a shaft or an irrigation apparatus (60) of the catheter(22).

10. The method according to claim 8 part (ii), wherein the force comprises a contact force applied between the member and tissue of an organ, wherein, in response to applying the contact force, the member is configured to deform for conforming with a shape of the tissue.

## Patentansprüche

1. System (20), umfassend:
einen Katheter (22), der eine expandierbare distale Endanordnung (EDEA) (40) umfasst, die aufweist: (i) einen Sender (88), der mit der EDEA (40) gekoppelt und zum Senden eines ersten Signals konfiguriert ist, und (ii) eine Vielzahl von Empfängern (99), die mit einer elastischen Komponente der EDEA (40) gekoppelt und zum Erzeugen eines oder mehrerer jeweiliger zweiter Signale als Reaktion auf den Empfang des ersten Signals konfiguriert sind;
wobei eine der folgenden Bedingungen erfüllt ist:
(i) die EDEA (40) umfasst einen Korb und ein oder mehrere der Vielzahl von Empfängern (99) sind mit jeder Rippe (55) des Korbs gekoppelt; oder
(ii) die EDEA (40) umfasst einen Ballon und die Vielzahl von Empfängern (99) ist an mehreren Positionen auf der Außenfläche des Ballons angeordnet;
und wobei das System ferner umfasst:
einen Prozessor (42), der konfiguriert ist zum: (i) Halten eines Kalibrierungsdatensatzes zum Quantifizieren einer Beziehung zwischen mindestens einem der jeweiligen zweiten Signale und einem Deformationsgrad mindestens einer der Rippen (55) oder eines Elements des Ballons, und (ii) Schätzen der Kontaktkraft basierend auf mindestens einem der jeweiligen zweiten Signale und dem Kalibrierungssatz.

2. System (20) nach Anspruch 1, Teil (i), wobei die Kraft eine Kontaktkraft umfasst, die zwischen der EDEA (40) und dem Gewebe eines Organs ausgeübt wird, und wobei als Reaktion auf die Ausübung der Kontaktkraft mindestens eine der Rippen (55) konfiguriert ist, um sich zu verformen, um sich der Form des Gewebes anzupassen.

3. System (40) nach Anspruch 2, wobei der Sender (88) mit einer starren Komponente der EDEA (40) gekoppelt ist.

4. System (40) nach Anspruch 3, wobei die starre Komponente eine Spüleinrichtung (60) oder einen Schaft des Katheters umfasst.

5. System (20) nach Anspruch 1, Teil (i), wobei der Prozessor (42) konfiguriert ist, um den Kalibrierungssatz vor dem Einsetzen der EDEA (40) in das Organ zu erstellen und die Kontaktkraft abzuschätzen, wenn die EDEA (40) in Kontakt mit dem Gewebe gebracht wird.

6. System (20) nach Anspruch 2, wobei, wenn sich die EDEA (40) in einer expandierten Position befindet, wobei das eine oder die mehreren jeweiligen zweiten Signale Folgendes umfassen: (i) ein erstes gegebenes Signal, das von einem gegebenen Empfänger (99) des einen oder der mehreren Empfänger (99) empfangen wird, ohne dass die Kontaktkraft angewendet wird, und (ii) ein zweites gegebenes Signal, das von dem gegebenen Empfänger (99) empfangen wird, wenn die Kontaktkraft angewendet wird, und wobei der Prozessor konfiguriert ist, um die Kontaktkraft basierend auf dem ersten und dem zweiten gegebenen Signal zu schätzen.

7. System (20) nach Anspruch 1, Teil (ii), wobei die elastische Komponente ein Element des Ballons umfasst.

8. Verfahren zum Herstellen eines Katheters (22), das Verfahren umfassend:
Koppeln eines Senders (88) zum Übertragen eines ersten Signals an eine expandierbare distale Endanordnung (EDEA) (40) eines Katheters (22);
Koppeln einer Vielzahl von Empfängern (99) an eine elastische Komponente der EDEA zum Erzeugen eines oder mehrerer jeweiliger zweiter Signale als Reaktion auf den Empfang des ersten Signals; und
elektrisches Koppeln der Vielzahl von Empfängern (99) an einen Prozessor (42) zum Empfangen des einen oder der mehreren jeweiligen zweiten Signale und zum Schätzen einer auf die elastische Komponente ausgeübten Kraft basierend auf dem einen oder den mehreren jeweiligen zweiten Signalen;
wobei eine der folgenden Bedingungen erfüllt ist:
(i) die EDEA (42) umfasst einen Korb und ein oder mehrere der Vielzahl von Empfängern (99) sind mit jeder Rippe (55) des Korbs gekoppelt; oder
(ii) die EDEA umfasst einen Ballon und die Vielzahl von Empfängern (99) ist an mehreren Positionen auf der Außenfläche des Ballons angeordnet.

9. Verfahren nach Anspruch 8, Teil (i), wobei der Sender (88) mit einer starren Komponente der EDEA gekoppelt ist, und wobei die starre Komponente einen Schaft oder eine Spüleinrichtung (60) des Katheters (22) umfasst.

10. Verfahren nach Anspruch 8, Teil (ii), wobei die Kraft eine Kontaktkraft umfasst, die zwischen dem Element und dem Gewebe eines Organs ausgeübt wird, wobei das Element als Reaktion auf die Ausübung der Kontaktkraft konfiguriert ist, um sich zu verformen, um sich an die Form des Gewebes anzupassen.

## Revendications

1. Système (20), comprenant :
un cathéter (22), comprenant un ensemble extrémité distale expansible (EDEA) (40) ayant : (i) un transmetteur (88), qui est couplé à l'EDEA (40) et est configuré pour transmettre un premier signal, et (ii) une pluralité de récepteurs (99), qui sont couplés à un composant élastique de l'EDEA (40), et sont configurés pour produire un ou plusieurs seconds signaux respectifs en réponse à la réception du premier signal ;
dans lequel l'une des conditions suivantes est respectée :
(i) l'EDEA (40) comprend un panier et un ou plusieurs parmi la pluralité de récepteurs (99) sont accouplés à chaque cannelure (55) du panier ; ou
(ii) l'EDEA (40) comprend un ballonnet et la pluralité de récepteurs (99) sont disposés au niveau de plusieurs positions sur la surface externe du ballonnet ;
et dans lequel le système comprend en outre :
un processeur (42) qui est configuré pour : (i) maintenir un ensemble de données d'étalonnage permettant de quantifier une relation entre au moins l'un des seconds signaux respectifs et un niveau de déformation d'au moins l'une des cannelures (55) ou d'un élément du ballonnet, et (ii) d'estimer la force de contact en fonction d'au moins l'un parmi les seconds signaux respectifs et l'ensemble d'étalonnage.

2. Système (20) selon la revendication 1 partie (i), dans lequel la force comprend une force de contact appliquée entre l'EDEA (40) et un tissu d'un organe, et dans lequel, en réponse à l'application de la force de contact, au moins l'une des cannelures (55) est conçue pour se déformer pour épouser la forme du tissu.

3. Système (40) selon la revendication 2, dans lequel le transmetteur (88) est accouplé à un composant rigide de l'EDEA (40).

4. Système (40) selon la revendication 3, dans lequel le composant rigide comprend un appareil d'irrigation (60) ou une tige du cathéter.

5. Système (20) selon la revendication 1 partie (i), dans lequel le processeur (42) est configuré pour produire l'ensemble d'étalonnage avant insertion de l'EDEA (40) dans l'organe, et pour estimer la force de contact lorsque l'EDEA (40) est placé en contact avec le tissu.

6. Système (20) selon la revendication 2, dans lequel, lorsque l'EDEA (40) est dans une position en expansion, le ou les seconds signaux respectifs comprennent : (i) un premier signal donné reçu d'un récepteur (99) donné parmi le ou les récepteurs (99) sans application de la force de contact, et (ii) un second signal donné reçu du récepteur (99) donné lorsque la force de contact est appliquée, et dans lequel le processeur est configuré pour estimer la force de contact en fonction des premier et second signaux donnés.

7. Système (20) selon la revendication 1 partie (ii) dans lequel le composant élastique comprend un élément du ballonnet.

8. Procédé permettant de produire un cathéter (22), le procédé comprenant :
l'accouplement, à un ensemble extrémité distale expansible (EDEA) (40) d'un cathéter (22), d'un transmetteur (88) permettant de transmettre un premier signal ;
l'accouplement, à un composant élastique de l'EDEA, d'une pluralité de récepteurs (99) permettant de produire un ou plusieurs seconds signaux respectifs en réponse à la réception du premier signal ; et
le couplage électrique de la pluralité de récepteurs (99) à un processeur (42) permettant de recevoir le ou les seconds signaux respectifs et permettant d'estimer, en fonction du ou des seconds signaux respectifs, une force appliquée au composant élastique ;
dans lequel l'une des conditions suivantes est respectée :
(i) l'EDEA (42) comprend un panier et un ou plusieurs parmi la pluralité de récepteurs (99) sont accouplés à chaque cannelure (55) du panier ; ou
(ii) l'EDEA comprend un ballonnet et la pluralité de récepteurs (99) sont disposés au niveau de plusieurs positions sur la surface externe du ballonnet.

9. Procédé selon la revendication 8 partie (i), dans lequel le transmetteur (88) est accouplé à un composant rigide de l'EDEA, et dans lequel le composant rigide comprend une tige ou un appareil d'irrigation (60) du cathéter (22).

10. Procédé selon la revendication 8 partie (ii), dans lequel la force comprend une force de contact appliquée entre l'élément et un tissu d'un organe, dans lequel, en réponse à l'application de la force de contact, l'élément est conçu pour se déformer pour épouser la forme du tissu.
